(19) Europäisches Patentamt
European Patent Office
Office européen des brevets

(11) Publication number: **0 160 284**
**B1**

# (12) EUROPEAN PATENT SPECIFICATION

(45) Date of publication of patent specification: **24.10.90**

(21) Application number: **85105112.8**

(22) Date of filing: **26.04.85**

(51) Int. Cl.$^5$: **C 07 D 498/06**, C 07 D 498/16 // A61K31/535

## (54) Quino-benoxazine antibacterial compounds.

(30) Priority: **26.04.84 US 604347**
**26.04.84 US 604191**
**26.04.84 US 604198**

(43) Date of publication of application:
**06.11.85 Bulletin 85/45**

(45) Publication of the grant of the patent:
**24.10.90 Bulletin 90/43**

(84) Designated Contracting States:
**BE CH DE FR GB IT LI NL SE**

(56) References cited:
**DD-A- 203 719**
**US-A-4 382 892**

**PATENT ABSTRACTS OF JAPAN, unexamined applications, section C, vol. 6, no. 174, 08 Sep 1982, The Patent Office Japanese Govt.; p. 76 C 123**

(73) Proprietor: **ABBOTT LABORATORIES**
**14th Street and Sheridan Road North St**
**North Chicago Illinois 60064 (US)**

(72) Inventor: **Chu, Daniel Tim-Wo**
**204 Ashland Court**
**Vernon Hills Illinois 60061 (US)**

(74) Representative: **Modiano, Guido et al**
**MODIANO, JOSIF, PISANTY & STAUB Modiano & Associati Via Meravigli, 16**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 160 284 B1

## Description

This invention relates to new quino-benoxazine derivatives having antibacterial properties and compositions containing the new quino-benoxazine derivatives useful for treating mammalian patients.

It is known that certain quinoline compounds exhibit antibacterial properties, notably certain 7-piperazinyl-4-oxo-1,4-dihydroquinoline-3-carboxylic acid derivatives which are substituted in the 1 position with an alkyl, benzyl or acetyl substituent. U.S. patent No. 4,292,317 discloses derivatives of 7-piperazinyl-4-oxo-1,4-dihydroquinoline-3-carboxylic acids wherein the 1 position is substituted by an alkyl group or a vinyl group. In U.S. Patent No. 4,284,629 there are disclosed various 4-oxo-1,4-dihydroquinoline-3-carboxylic acids in which the 1 position is substituted with a cycloalkyl group.

This invention relates to novel antibacterial agents having the formula:

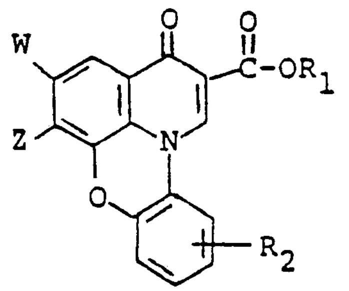

(IA)

wherein $R_2$ is one or more groups selected from hydrogen, halogen, $C_1$ to $C_6$ alkyl including substituted derivatives thereof, wherein the substituents are selected from hydroxy and halo; nitro, carboxyl, cyano, methylenedioxy, a group having the formula —Y—$R_3$ wherein —Y— is —O— or —S— and $R_3$ is hydrogen or $C_1$ to $C_6$ alkyl and an amine having the formula:

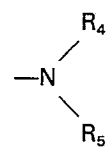

wherein $R_4$ and $R_5$ are each independently hydrogen or $C_1$ to $C_6$ alkyl.

$R_1$ is hydrogen or a carboxy-protecting group.

Z is an amino group having the formula:

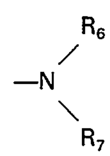

wherein $R_6$ is hydrogen or $C_1$ to $C_{10}$ alkyl or substituted derivatives thereof wherein the substituents are selected from hydroxy and halo; and $R_7$ is selected from the groups consisting of $C_1$ to $C_{10}$ alkyl and substituted derivatives thereof wherein the substituents are selected from hydroxy and halo and furthermore consisting of an amino group, a mono-($C_1$ to $C_6$) alkylamino group or a di-($C_1$ to $C_6$) alkylamino group.

Alternatively, Z can be a 4-pyridyl group substituted or unsubstituted, suitable substituents including $C_1$ to $C_6$ alkyl, halogen, a group of the formula —Y—$R_{12}$ wherein Y is —O— or —S— and $R_{12}$ is loweralkyl, hydroxy, alkanoyl containing 1 to 6 carbon atoms, alkanoylamido containing 1 to 6 carbon atoms and an amine of the formula:

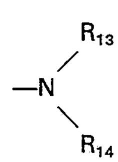

wherein $R_{13}$ and $R_{14}$ are each independently selected from the group consisting of hydrogen, $C_1$ to $C_6$ alkyl and substituted derivatives thereof, wherein the substituents are selected from hydroxy and halo.

Illustrative of compounds of the invention where Z is a substituted or unsubstituted pyridyl group are those in which Z is 4-pyridyl; $R_2$ is a hydrogen, fluoro, difluoro or methylenedioxy group; $R_1$ is hydrogen; and W is fluoro or hydrogen.

Alternatively, Z can be an aliphatic heterocyclic ring having the formula

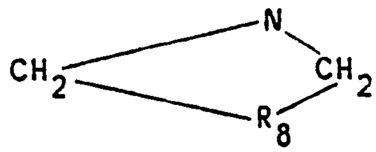

wherein $R_8$ is selected from the group consisting of dimethylene and a group of the formula

$$-CH_2-R_9-CH_2-$$

wherein $R_9$ is selected from the group consisting of —S—, —O—, —NH—, $—CH_2—$ and $—CH_2—NH—$. Also included are substituted derivatives of such heterocyclic rings wherein the substituent is one or more of a $C_1$ to $C_6$ alkyl group, hydroxy, alkanoyl containing 1 to 6 carbon atoms, alkanoylamido containing 1 to 6 carbon atoms, and an amino group having the formula:

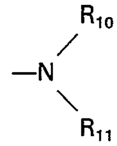

wherein $R_{10}$ and $R_{11}$ are each independently selected from the group consisting of hydrogen, $C_1$ to $C_6$ alkyl and hydroxy- and halo-substituted $C_1$ to $C_6$ alkyl.

Illustrative of such heterocyclic groups are piperazinyl groups, 4-acylpiperazinyl groups, (4-methyl)-piperazinyl, (3,5-dimethyl)-piperazinyl, piperidinyl groups, pyrrolidinyl groups, (3-amino)-pyrrolidinyl, (3-methylamino)-pyrrolidinyl, (3-dimethyl-amino)-pyrrolidinyl, (3-acetamido)-pyrrolidinyl, (3-methyl-acetamido)-pyrroldinyl, (3-hydroxy)-pyrrolidinyl, morpholino groups, thiomorpholino groups and homopiperazinyl groups (i.e., hexahydro-1-H-1,4-diazepinyl).

W is halogen or hydrogen or, alternatively, W and Z together can form a methylenedioxy bridge.

Illustrative of compounds of the invention where W and Z together form a methylenedioxy bridge are those in which $R_2$ is a hydrogen, $C_1$ to $C_6$ alkyl, 10-fluoro, methylenedioxy or 8,10-difluoro group; and $R_1$ is hydrogen.

As used herein, the term "halogen" refers to chloro, bromo, fluoro and iodo groups, while the term "$C_1$ to $C_6$ alkyl" refers to loweralkyl groups including methyl, ethyl, propyl, isopropyl and butyl.

As used herein, the term $C_1$ to $C_6$ alkyl and substituted derivatives thereof refers to hydroxy and halo-substituted derivatives of $C_1$ to $C_6$ alkyl. Such groups include, for example, a chloromethyl group, a chloroethyl group, a chloropropyl group, a hydroxyethyl group, and a trifluoromethyl group.

$R_2$ can also be a group of the formula $—Y—R_3$. Representative groups of this type include a hydroxy group, a mercapto group, a lower alkoxy group, such as methoxy, ethoxy, propoxy, as well as the thio analogs thereof, namely a methylmercapto group, and an ethylmercapto group.

As used herein, the term "carboxy-protecting group" refers to and includes the residue of a carboxylic acid ester group. Such carboxy-protecting groups are well known to those skilled in the art, having been extensively used in the protection of carboxyl groups in the penicillin and cephalosporin fields, as described in U.S. Patent Nos. 3,840,556 and 3,719,667.

In general, such carboxy-protecting groups can be relatively easily cleaved to yield the corresponding free carboxy group. Representative protecting groups include $C_1$—$C_8$ alkyl (e.g., methyl, ethyl, tertiary butyl), substituted alkyl (e.g., dimethylaminoethyl), benzyl and substituted derivatives thereof such as alkoxy and nitrobenzyl groups; also suitable are acyl groups such as pivaloyloxymethyl groups.

Also included within the scope of the present invention are pharmaceutically acceptable salts of the foregoing compounds. As used herein, the term "pharmaceutically acceptable salts" refers to nontoxic acid addition salts and alkaline earth metal salts of the compounds of Formula IA. The salts can be prepared *in situ* during the final isolation and purification of the compounds of formula IA, or separately by reacting the free base or acid functions with a suitable organic acid or base. Representative acid addition salts include the hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, succinate, tartrate, glucoheptonate, lactobionate, lauryl sulfate salts and the like. Representative alkali or alkaline earth metal salts include the sodium, calcium, potassium and magnesium salts. It has been found that the compounds of the present invention possess antibacterial activity against a wide spectrum of gram positive and gram negative bacteria, as well as enterobacteria. The compounds of the invention are therefore useful in the antibiotic treatment of susceptible bacterial infections in both humans and animals. In addition, the compounds, by reason of their *in vitro* activity, may be used in scrub solutions for surface inhibition of bacterial growth.

Susceptible organisms generally include those gram positive and gram negative, aerobic and anaerobic organisms whose growth can be inhibited by the compounds of the invention such as *Staphylococcus, Lactobacillus, Streptococcus, Sarcina, Escherichia, Enterobacter, Klebsiella, Pseudomonas, Acinetobacter, Proteus, Citrobacter, Nisseria, Baccillus, Bacteroides, Peptococcus, Clostridium, Salmonella, Shigella, Serratia, Haemophilus, Brucella,* and other organisms. In addition to exhibiting highly effective antibacterial activity, the compounds of the invention exhibit increased and improved solubility characteristics as compared with prior quinoline-3-carboxylic acid compounds in the art.

The compounds of Formula IA may also be formulated into compositions together with pharmaceutically acceptable carriers for parenteral injection, for oral administration in solid or liquid form, for rectal administration, and the like.

Compositions according to the invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixers containing inert diluents commonly used in the art, such as water. Besides such inert diluents, compositions can also include adjuvants, such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve antibacterial activity in accordance with the desired method of administration. The selected dosage level therefore depends upon the nature of the active compound administered, the route of administration, the desired duration of treatment and other factors. Generally, daily dosage levels of the compounds of Formula I of about 0.1 to about 750, more preferably about 0.25 to about 500 and most preferably about 0.5 to about 300 mg of active ingredient per kg of body weight are effective when administered orally to a mammalian patient suffering from an infection caused by a susceptible organism. If desired, the daily dose may be divided into multiple doses for administration, e.g., two to four times per day.

Compounds according to the invention wherein W is F can be prepared by the reaction illustrated below:

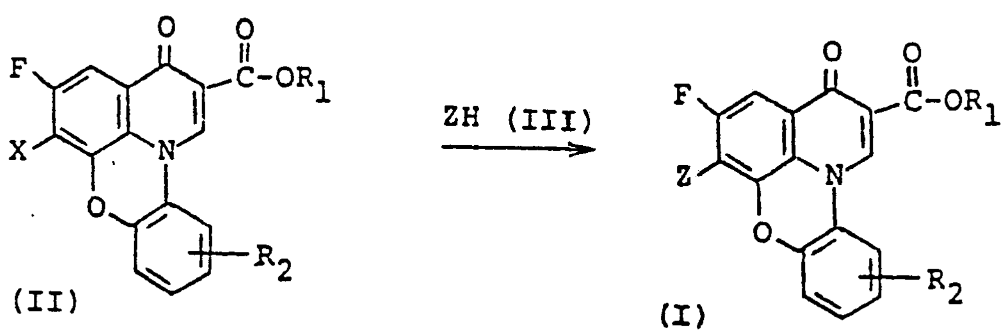


(II) (I)

wherein X is a halogen and $R_2$ and Z are the same as described above with the exclusion of pyridine.

The reaction may be performed by heating a compound of the formula (II) with an amine of formula (III) at a temperature of from 20°C to 200°C, and preferably from 70°C to 150°C, in the presence of a suitable organic polar solvent such as dimethylsulfoxide, sulfolane, dimethylformamide, dimethylacetamide, 1-methyl-2-pyrrolidinone or water. It is desirable to carry out the reaction in the presence of an acid-acceptor such as triethylamine, potassium carbonate and the like at a molar ratio of 1.0 to 1.2 mole of the acid-acceptor per mole of the compound of the formula (II). The amine (III) can also be used as acid acceptor in which 2 or more molar excess of this reagent is used. The compounds of the formula (II) may be prepared in accordance with the following reaction scheme, in which $R_2$ is as described above, and X can be independently identical or nonidentical halogen:

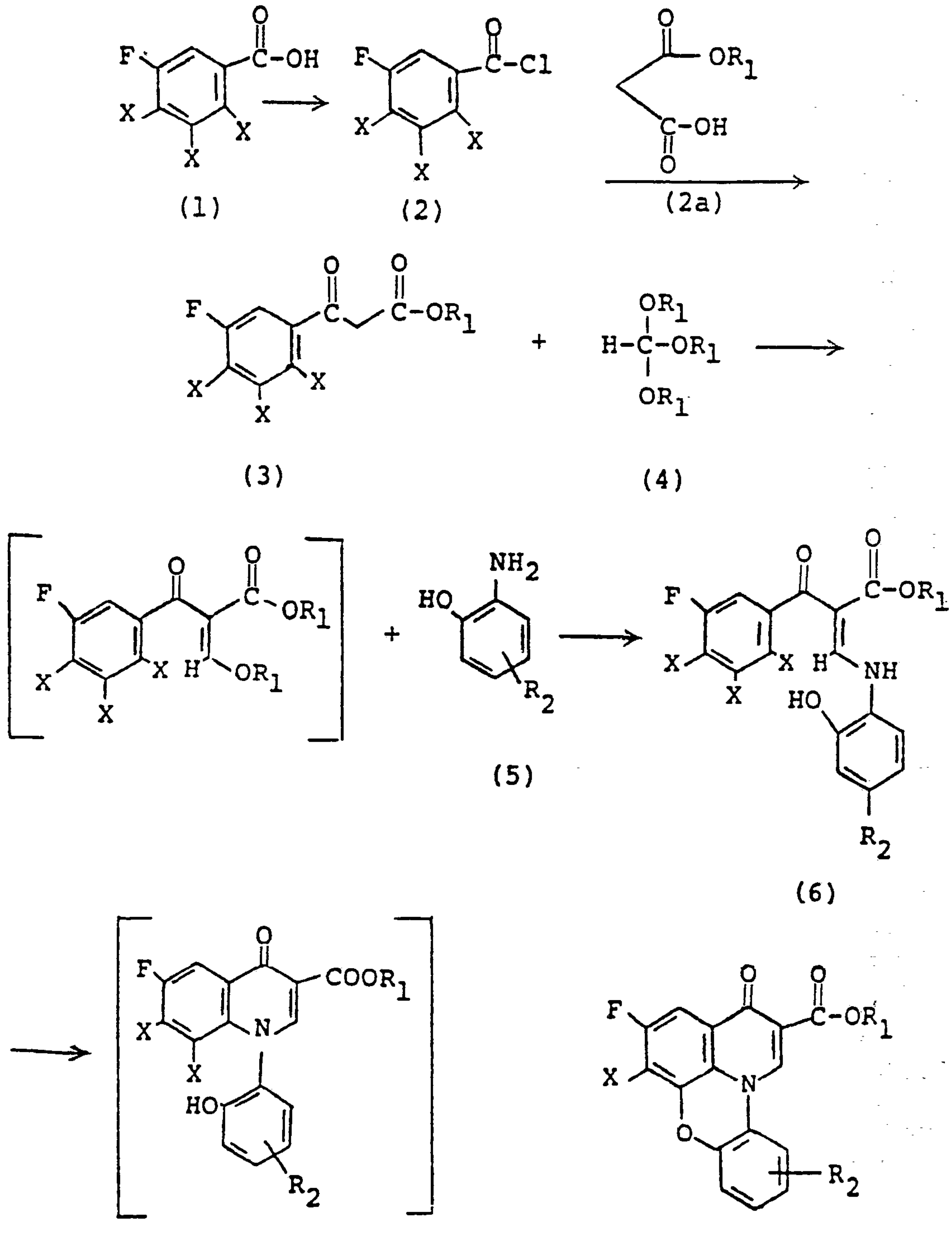

(1) (2) (2a) (3) (4) (5) (6) (6a) (II)

In accordance with the foregoing reaction scheme, the 2,3,4-trihalo-5-fluorobenzoic acid (1) is treated with thionyl chloride to produce the corresponding acid chloride (2). Displacement of the acid chloride (2) with malonic acid half ester (2a) in the presence of n-butyl lithium yields the β-ketoester (3).

The β-ketoester (3) is then treated with a trialkylorthoformate (4) in the presence of an acid anhydride, preferably acetic anhydride, followed by reaction with substituted or unsubstituted O-hydroxyaniline (5) to obtain the enaminoketoester (6). In the trialkylorthoformate (4), $R_1$ may be an alkyl group of, for example, from 1 to 10 carbon atoms, but is preferably loweralkyl, such as ethyl. Reaction with the trialkylorthoformate is preferably conducted at elevated temperatures, such as from about 50°C to about 150°C, preferably from about 100°C to about 140°C, to obtain an oily liquid, which may be isolated or unisolated, as desired (shown in brackets in the reaction scheme). Reaction of the latter with the substituted or unsubstituted O-hydroxyaniline (5) is preferably conducted in an appropriate aprotic or nonaprotic solvent,

preferably methylene chloride or tetrahydrofuran, and may be conducted at room or suitable elevated temperature, as desired.

The enaminoketoester (6) is then cyclized, such as by treatent with a strong base as defined above, preferably sodium hydride, to obtain the 1-chloro-2-fluoro-4-oxo-4H-quino[2,3,4-i,j][1,4]benoxazine-5-carboxylic acid ester (II) ($R_1$ = alkyl) through the intermediate (6a). Cyclization is conducted in the presence of an aprotic solvent, such as dimethoxyethane, bis(2-methoxyethyl)ether, dimethylformamide, tetrahydrofuran or chlorobenzene, and is preferably conducted at temperatures of about 20°C to about 145°C, more preferably at the reflux temperature of the solvent employed.

The ester (II) is subjected to hydrolysis, such as by treatment with sodium hydroxide, or diluent mineral acid to form the free acid (II) ($R_1$ = H).

The 4-oxo-4H-quino[2,3,4-i,j][1,4]benoxazine-5-carboxylic acid (I) ($R_1$ = H) can then be converted into the corresponding ester (I) ($R_1$ H), if desired, by conventional esterification procedures, such as by treating the free acid (I) ($R_1$ = H) with the appropriate alcohol in the presence of an acid catalyst, by converting the free acid (I) ($R_1$ = H) into the corresponding acid chloride followed by displacement of the chloro radical with the appropriate alcohol, or by treating the sodium salt of the acid (I) ($R_1$ = H) with a suitable reactive halide, such as chloromethylpivalate or dimethylaminoethyl chloride in dimethoxyethane to obtain, for example, the pivaloyloxymethyl ester (I) wherein $R_1$ is $—CH_2OCOC(CH_3)_3$ or dimethylaminoethyl ester (I) wherein $R_1$ is $CH_2CH_2N(CH_3)_2$.

The foregoing may be better understood from the following examples, which are presented for purposes of illustration and are not intended to limit the scope of the inventive concepts. As used in the following examples, the references to compounds, such as (1), (2), (3), etc., and to substituents, such as R, $R_1$, $R_2$, etc., refer to the corresponding compounds and substituents in the foregoing reaction scheme and in formulae I and II.

Example 1

1-(1-piperazinyl)-2-fluoro-4-oxo-4H-quino[2,3,4-i,j][1,4]benoxazine-5-carboxylic acid

(a) To a solution of 3.9 g of 2,3,4,5-tetrafluorobenzoic acid in 20 ml of methylene chloride is added 4 ml of thionyl chloride. After refluxing for 30 minutes, the reaction mixture is evaporated to dryness to give 4.95 g of the acid chloride.

This is slowly added to a dry ice-cooled solution of 3.5 g of malonic acid monoethyl ester in 35 ml of tetrahydrofuran, 37.9 ml of 1.4 M n-butyl lithium in THF, and the flask is warmed to −5°C for 5 minutes and then is cooled back to −70°C. To the resulting solution there is added 4.95 g of the acid chloride in 10 ml of THF. The cooling bath is removed, and the solution allowed to warm to room temperature after an hour. The solution is then partitioned between 1 N HCl and ether. The ether portion is washed with $NaHCO_3$ and dried over $MgSO_4$, and then evaporated to obtain the liquid β-ketoester (3).

(b) A solution of 20 g of the above β-ketoester (3) in 18.5 ml of triethylorthoformate and 45 ml of acetic anhydride is treated at 135°C for 1½ hours with the removal of the ethyl acetate formed during the reaction. The solution is evaporated under reduced pressure to a mobile oil. The oil is then dissolved in 200 ml of methylene chloride and 9.5 g of o-hydroxyaniline is added into the solution. After 1 hour, the solution is evaporated to dryness and crystallized from 200 ml of hexane and 5 ml of ether yielding (6) wherein $R_2$ = $C_2H_5$, $R_2$ = H, X = F).

(c) To a cold solution of 15 g of the preceding product (6), $R_1$ = $C_2H_5$, X = F, $R_2$ = H in 150 ml dimethoxyethane (DME) is slowly added 3.33 g of a 60% sodium hydride-in-oil suspension. The mixture is refluxed for 24 hours and is cooled and diluted with water to a volume of 1.5 liters. The mixture is then filtered and the solid is washed with a 1:1 hexane/ether solution to obtain (II), ($R_1$ = $C_2H_5$, X = F, $R_2$ = H).

(d) To a suspension of 7 g of (II) ($R_1$ = $C_2H_5$, X = F, $R_2$ = H) in 30 ml THF is added a sodium hydroxide solution (0.91 g) in 20 ml water. The mixture is heated at 80°C for 1 hour resulting in a clear solution which is evaporated under reduced pressure to dryness. The solid is dissolved in 200 ml water and 2.5 ml acetic acid is added. The resulting precipitate is filtered and washed with cold water, crystallized from dimethylformamide (DMF) to produce 1,2-difluoro-4-oxo-4H-quino[2,3,4-i,j][1,4]-benzoxazine-5-carboxylic acid (II) ($R_1$ = H, X = F, $R_2$ = H).

(e) To a solution of 2.7 g of 1,2-difluoro-4-oxo-4H-quino[2,3,4-i,j][1,4]benoxazine-5-carboxylic acid (II), ($R_1$ = H, X = F, $R_2$ = H) in 15 ml of 1-methyl-2-pyrrolidinone at 115°C is added 3 ml piperazine. After stirring at 100°C for 20 hours, the solvent is removed by reduced pressure to dryness. Ethanol is added to the residue and the resulting mixture is filtered and washed with ether and then washed with very small amounts of cold water to give (I)

($R_1$=H, $R_2$=H, Z=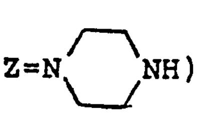).

The resulting dried solid (I) is suspended in 30 ml water and 8.5 ml. 1N HCl is added to and warmed to dissolve. Removal of the solvent under reduced pressure gives hydrochloride salt of 1-(1-piperazinyl)-2-fluoro-4-oxo-4H-quino[2,3,4-i,j][1,4]benoxazine-5-carboxylic acid (I)

($R_1$=H, $R_2$=H, Z=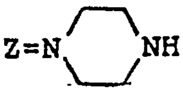).

To the hydrochloride salt is added one molar equivalent of an aqueous solution of sodium hydroxide, and the resulting precipitate is filtered to obtain 1-(1-pierazinyl)-2-fluoro-4-oxo-4H-quino[2,3,4-i,j][1,4]-benoxazine-5-carboxylic acid (I).

(f) Alternately, the title compound is prepared as follows: To a suspension of 5 g of compound (II) (product of 1(d) in 40 ml 1-methyl-2-pyrrolidinone at 120°C under nitrogen atmosphere is added 9.5 ml of N-carboethoxypiperazine. After 20 hours, the solvent is removed under reduced pressure and the residue is suspended in 150 ml ethanol and refluxed for ½ hour. The reaction mixture is then cooled and filtered. The resulting solid is washed with cold ethanol and water to obtain compound (I)

($R_1$=H, $R_2$=H, Z=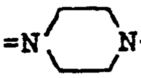-$COOC_2H_5$)

To a suspension of 5 g of the preceding compound (I)

($R_1$=H, $R_2$=H, Z=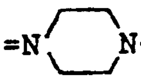-$COOC_2H_5$)

in 25 ml of ethanol at 80°C is added 50 ml of 10% NaOH solution. The solution is heated at 80°C for 6 hours. The solvent is removed and the solid is dissolved in 100 ml water. The pH of the solution is adjusted to pH 7 by the addition of 10% acetic acid. The precipitate is filtered and washed with cold water yielding 1-(1-piperazinyl)-2-fluoro-4-oxo-4H-quino[2,3,4-i,j][1,4]benoxazine-5-carboxylic acid (I)

($R_1$=H, $R_2$=H, Z=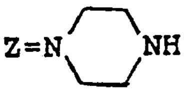).

Example 2

1-(1-(4-methyl)piperazinyl)-2-fluoro-4-oxo-4H-quino[2,3,4-i,j][1,4]benoxazine-5-carboxylic acid

The procedure of Example 1 is repeated replacing piperazine in Example 1(e) with N-methylpiperazine to obtain 1-(1-(4-methyl)piperazinyl)-2-fluoro-4-oxo-4H-quino[2,3,4-i,j][1,4]benoxazine-5-carboxylic acid (I)

($R_1$=H, $R_2$=H, Z=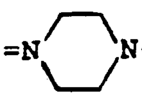-$CH_3$)

and its hydrochloride salt.

Example 3

1-(1-pyrrolidinyl)-2-fluoro-4-oxo-4H-quino[2,3,4-i,j][1,4]benoxazine-5-carboxylic acid

In the described fashion as Example 1, replacing piperazine in Example 1(e) with pyrrolidine, one can obtain 1-(1-pyrrolidinyl)-2-fluoro-4-oxo-4H-quino[2,3,4-i,j][1,4]benoxazine-5-carboxylic acid (I)

($R_1$=H, $R_2$=H, Z=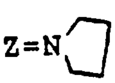 ).

Example 4

1-(1-3-hydroxypyrrolidinyl)-2-fluoro-4-oxo-4H-quino[2,3,4-i,j][1,4]benoxazine-5-carboxylic acid

The procedure of Example 1 can be repeated replacing piperazine in Example 1(e) with 3-hydroxypyrrolidine to obtain 1-(1-3-hydroxypyrrolidinyl)-2-fluoro-4-oxo-4H-quino[2,3,4-i,j][1,4]benoxazine-5-carboxylic acid (I)

($R_1$=$R_2$=H, Z-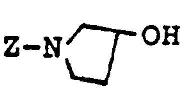 ).

Example 5

1-(1-3-aminopyrrolidinyl)-2-fluoro-4-oxo-4H-quino[2,3,4-i,j][1,4]benoxazine-5-carboxylic acid

(a) In the described fashion as Example 1 replacing piperazine in Example 1(e) with 3-acetamido-pyrrolidine, one can obtain 1-(1-3-acetamidopyrrolidinyl)-2-fluoro-4-oxo-4H-quino[2,3,4-i,j][1,4]-benoxazine-5-carboxylic acid (I)

($R_1$=$R_2$=H, Z=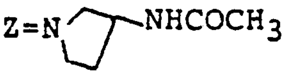 ).

(b) The product of the above reaction can be hydrolyzed with hydrochloric acid at 80°C to give 1-(1-3-aminopyrroldinyl)-2-fluoro-4-oxo-4H-quino[2,3,4-i,j][1,4]benoxazine-5-carboxylic acid (I)

($R_1$=$R_2$=H, Z=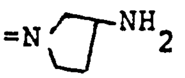 )

and its hydrochloride salt.

Example 6

1-(1-3-methylaminopyrrolidinyl)-2-fluoro-4-oxo-4H-quino[2,3,4-i,j][1,4]benoxazine-5-carboxylic acid

(a) In the described fashion as Example 1 replacing piperazine in Example 1(e) with 3-N-formyl-N-methylpyrrolidinyl, one can obtain 1-(1,3-N-formyl-N-methylaminopyrrolidinyl)-2-fluoro-4-oxo-4H-quino-[2,3,4-i,j][1,4]benzoxazine-5-carboxylic acid (I)

($R_1$=$R_2$=H, Z=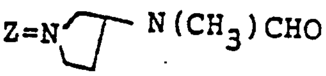).

(b) The product of the above reaction can then be hydrolyzed with hydrochloric acid at 80°C to give 1-(1-3-methylaminopyrrolidinyl)-2-fluoro-4-oxo-quino[2,3,4-i,j][1,4]benoxazine-5-carboxylic acid (I),

($R_1$=$R_2$=H, Z= 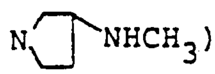)

and its hydrochloride salt.

Example 7

1-(1,-3-dimethylaminopyrrolidinyl)-2-fluoro-4-oxo-4H-quino[2,3,4-i,j][1,4]benoxazine-5-carboxylic acid

The procedure of Example 1 is repeated replacing piperazine in Example 1(e) with 3-dimethylaminopyrrolidine to obtain 1-(1-3-dimethylaminopyrrolidinyl)-2-fluoro-4-oxo-4H-quino[2,3,4-i,j][1,4]benoxazine-5-carboxylic acid (I) and its hydrochloride salt

($R_1$=$R_2$=H, Z=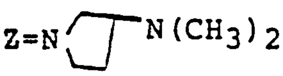 )..

Example 8

1-(1-piperidinyl)-2-fluoro-4-oxo-4H-quino[2,3,4-i,j][1,4]benoxazine-5-carboxylic acid

The procedure of Example 1 can be repeated replacing piperazine in Example 1(e) with piperidine to obtain 1-(1-piperidinyl)-2-fluoro-4-oxo-4H-quino[2,3,4-i,j][1,4]benoxazine-5-carboxylic acid (I)

($R_1$=$R_2$=H, Z=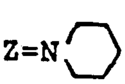 ).

Example 9

1-(4-morpholinyl)-2-fluoro-4-oxo-4H-quino[2,3,4-i,j][1,4]benoxazine-5-carboxylic acid

In the described fashion as Example 1, replacing piperazine in Example 1(e) with morpholine, one can obtain 1-(4-morpholinyl)-2-fluoro-4-oxo-4H-quino[2,3,4-i,j][1,4]benoxazine-5-carboxylic acid (I)

($R_1$=$R_2$=H, Z=-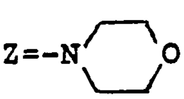).

Example 10

1-(4-thiomorpholinyl)-2-fluoro-4-oxo-4H-quino[2,3,4-i,j][1,4]benoxazine-5-carboxylic acid

The procedure of Example 1 can be repeated replacing piperazine in Example 1(e) with thiomorpholine to obtain 1-(4-thiomorpholinyl)-2-fluoro-4-oxo-quino[2,3,4-i,j][1,4]benoxazine-5-carboxylic acid (I)

($R_1$=$R_2$=H, Z=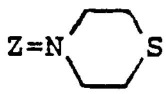).

Example 11

1-(1-3,5-dimethylpiperazinyl)-2-fluoro-4-oxo-4H-quino[2,3,4-i,j][1,4]benoxazine-5-carboxylic acid

In the described fashion as Example 1, replacing piperazine in Example 1(e) with 2,6-dimethylpiperazine, one obtains 1-(1-3,5-dimethylpiperazinyl)-2-fluoro-4-oxo-4H-quino[2,3,4-i,j][1,4]benoxazine-5-carboxylic acid (I) and its hydrochloride salt.

($R_1$=$R_2$=H, Z=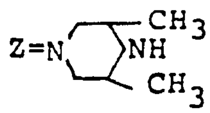).

Example 12

1-(1-homopiperazinyl)-2-fluoro-4-oxo-4H-quino[2,3,4-i,j][1,4]benoxazine-5-carboxylic acid

The procedure of Example 1 is repeated replacing piperazine in Example 1(e) with homopiperazine to obtain 1-(1-homopiperazinyl)-2-fluoro-4-oxo-4H-quino[2,3,4-i,j][1,4]benoxazine-5-carboxylic acid (I) and its hydrochloride salt

($R_1$=$R_2$=H, Z=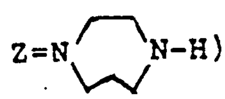).

Example 13

1-dimethylamino-2-fluoro-4-oxo-4H-quino[2,3,4-i,j][1,4]benoxazine-5-carboxylic acid

In the described fashion as Example 1, replacing piperazine in Example 1(e) with dimethylamine, one can obtain 1-dimethylamino-2-fluoro-4-oxo-4H-quino[2,3,4-i,j][1,4]benoxazine-5-carboxylic acid (I) ($R_1$ = $R_2$ = H, Z = $N(CH_3)_2$).

Example 14

1-(N-2-hydroxyethylamino)-2-fluoro-4-oxo-4H-quino[2,3,4-i,j][1,4]benoxazine-5-carboxylic acid

The procedure of Example 1 can be repeated replacing piperazine in Example 1(e) with N-2-hydroxyethylamine to obtain 1-(N-2-hydroxyethylamino)-2-fluoro-4-oxo-4H-quino[2,3,4-i,j][1,4]benoxazine-5-carboxylic acid (I) ($R_1$ = $R_2$ = H, Z = $NHC_2H_4$—OH).

Example 15

1-hydrazyl-2-fluoro-4-oxo-4H-quino[2,3,4-i,j][1,4]benoxazine-5-carboxylic acid

In the described fashion as Example 1, replacing piperazine in Example 1(e) with hydrazine, one can obtain 1-hydrazyl-2-fluoro-4-oxo-4H-quino[2,3,4-i,j][1,4]benoxazine-5-carboxylic acid ($R_1$ = $R_2$ = H, Z = $NHNH_2$).

Example 16

1-(1-piperazinyl)-2,10-difluoro-4-oxo-4H-quino[2,3,4-i,j][1,4]benoxazine-5-carboxylic acid

(a) In the described fashion as Example 1(b), replacing 2-hydroxyaniline with 2-hydroxy-4-fluoroaniline, one can obtain the enaminoketoester (6) ($R_1$ = $C_2H_5$, R = 10-fluoro, X = F).

(b) By following the Example 1(c) and 1(d), the preceding compound (6) can yield 1,2,10-trifluoro-4-oxo-4H-quino[2,3,4-i,j][1,4]benoxazine-5-carboxylic acid (II), ($R_1$ = H, X = F, $R_2$ = 10-fluoro).

(c) In the described fashion as Example 1(e), the above acid (II) can give the desired 1-(1-piperazinyl)-2,10-difluoro-4-oxo-4H-quino[2,3,4-i,j][1,4]benoxazine-5-carboxylic acid (I) and its hydrochloride salt

($R_1$=H, $R_2$=10-fluoro, Z=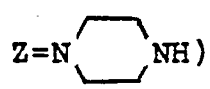).

Example 17

In the described fashion as Example 1(e), replacing the acid (II) ($R_1$ = $R_2$ = H, X = F) with the acid (II) of the product of Example 16(B) ($R_1$ = H, $R_2$ = 10-fluoro, X = F) and also replacing piperazine with an appropriate amine such as N-methylpiperazine, pyrrolidine, 3-hydroxypyrrolidine, 3-acetaminopyrrolidine, 3-N-formyl-N-methylaminopyrrolidine, 3-dimethylaminopyrrolidine, piperidine, morpholine, thiomorpholine, 2,6-dimethylpiperazine, homopiperazine, diethylamine and 2,2-dimethylhydrazine, one can obtain the following compounds:

(a) 1-(1,4-methylpiperazinyl)-2,10-difluoro-4-oxo-4H-quino[2,3,4-i,j][1,4]benoxazine-5-carboxylic acid (I) and its hydrochloride salt

($R_1$=H, $R_2$=10-fluoro, Z=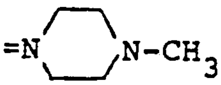).

(b) 1-(1-pyrrolidinyl)-2,10-difluoro-4-oxo-4H-quino[2,3,4-i,j][1,4]benoxazine-5-carboxylic acid (I)

($R_1$=H, $R_2$=10-fluoro, Z=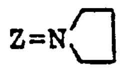).

(c) 1-(1-3-hydroxypyrrolidinyl)-2,10-difluoro-4-oxo-4H-quino[2,3,4-i,j][1,4]benoxazine-5-carboxylic acid (I)

($R_1$H, $R_2$=10-fluoro, Z=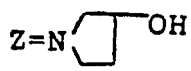).

(d) 1-(1-3-acetamidopyrrolidinyl)-2,10-difluoro-4-oxo-4H-quino[2,3,4-i,j][1,4]benoxazine-5-carboxylic acid (I)

($R_1$=H, $R_2$=10-fluoro, Z=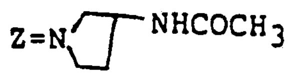).

(e) 1-(1-3-N-formyl-N-methylaminopyrrolidinyl)-2,10-difluoro-4-oxo-4H-quino[2,3,4-i,j][1,4]benoxazine-5-carboxylic acid (I)

($R_1$=H, $R_2$=10-fluoro, Z=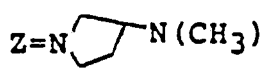).

(f) 1-(1-3-dimethylaminopyrrolidinyl)-2,10-difluoro-4-oxo-4H-quino[2,3,4-i,j][1,4]benoxazine-5-carboxylic acid (I)

($R_1$=H, $R_2$=10-fluoro, Z=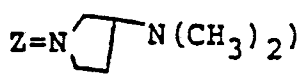)

and its hydrochloride salt.

(g) 1-(1-piperidinyl)-2,10-difluoro-4-oxo-4H-quino[2,3,4-i,j][1,4]benoxazine-5-carboxylic acid (I)

($R_1$H, $R_2$=10-fluoro, Z=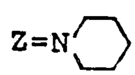).

(h) 1-(4-morpholinyl)-2,10-difluoro-4-oxo-4H-quino[2,3,4-i,j][1,4]benoxazine-5-carboxylic acid (I)

($R_1$=H, $R_2$=10-fluoro, Z=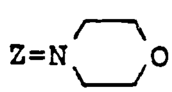).

(i) 1-(4-thiomorpholinyl)-2,10-difluoro-4-oxo-4H-quino[2,3,4-i,j][1,4]benoxazine-5-carboxylic acid (I)

($R_1$=H, $R_2$=10-fluoro, Z=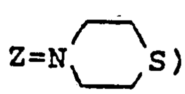).

(j) 1-(1,3,5-dimethylpiperazinyl)-2,10-difluoro-4-oxo-4H-quino[2,3,4-i,j][1,4]benoxazine-5-carboxylic acid (I)

($R_1$=H, $R_2$=10-fluoro, Z=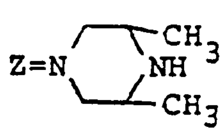)

and its hydrochloride salt.

(k) 1-(1-homopiperazinyl)-2,10-difluoro-4-oxo-4H-quino[2,3,4-i,j][1,4]benoxazine-5-carboxylic acid (I)

($R_1$H, $R_2$=10-fluoro, Z=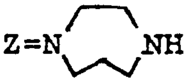)

and its hydrochloride salt.

(l) 1-diethylamino-2,10-difluoro-4-oxo-4H-quino[2,3,4-i,j][1,4]benoxazine-5-carboxylic acid (I) ($R_1$ = H, $R_2$ = 10-fluoro, Z = $N(CH_3)_2$).

(m) 1-N,N-dimethylhydrazyl-2,10-difluoro-4-oxo-4H-quino[2,3,4-i,j][1,4]benoxazine-5-carboxylic acid (I) ($R_1$ = H, $R_2$ = 10-fluoro, Z = $NHN(CH_3)_2$).

Example 18

In the described fashion of Example 5(b), the compounds of Examples 17(d) and 17(e) can give the following 2 compounds.

(a) 1-(1-3-aminopyrrolidinyl)-2,10-difluoro-4-oxo-quino[2,3,4-i,j][1,4]benoxazine-5-carboxylic acid (I) and its hydrochloride salt

($R_1$=H, $R_2$=1-fluoro, Z=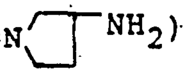).

(b) 1-(1-3-methylaminopyrrolidinyl)-2,10-difluoro-4-oxo-4H-quino[2,3,4-i,j][1,4]benoxazine-5-carboxylic acid (I) and its hydrochloride salt

($R_1$=H, $R_2$=10-fluoro, Z=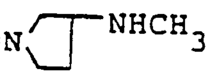).

Example 19

In the described fashion as Example 1(a-d), replacing o-hydroxyaniline with an appropriate substituted 2-hydroxyaniline

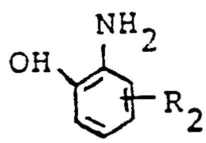

one can obtain the additional substituted 1,2-difluoro-4-oxo-4H-quino[2,3,4-i,j][1,4]benoxazine-5-carboxylic acid (II) as listed in Table I.

TABLE I

| Substituted 2-hydroxyaniline | Compound (II) ($R_1$ = H, X = F) obtained |
|---|---|
| $R_2$ 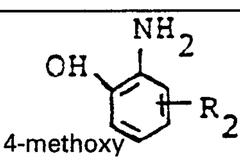 | $R_2$ |
| (a) 4-methoxy | 10-methoxy |
| (b) 4-methyl | 10-methyl |
| (c) 4-chloro | 10-chloro |
| (d) 4,6-difluoro | 8,10-difluoro |
| (e) 4,5-methylenedioxy | 9,10-methylenedioxy |
| (f) 4-hydroxy | 10-hydroxy |
| (g) 4-dimethylamino | 10-dimethylamino |
| (h) 5-fluoro | 9-fluoro |

Example 20

In the described fashion of Example 1(e), replacing the acid (II) ($R_1$ = H, $R_2$ = H, X = F) with the acid (II) of the compounds listed in Table I of Example 19 and also replacing piperazine with an appropriate amine such as N-methylpiperazine, pyrrolidine, 3-hydroxypyrrolidine, 3-acetamidopyrrolidine, 3-N-formyl-N-methylaminopyrrolidine, 3-dimethylaminopyrrolidine, piperidine, morpholine, thiomorpholine, 2,6-dimethylpiperazine, homopiperazine, dimethylamine and 2,2-dimethylhydrazine, and extra hydrolysis step if required as in Example 5(b), one can obtain the following additional compounds (I) as summarized in Table II.

TABLE II

| Piperazine replacement | Compound II used ($R_1$ = H, X = F) | Compound I obtained ($R_1$ = H) | |
|---|---|---|---|
| ZH | $R_2$ | $R_2$ | Z |
| 1. piperazine | 8,10-difluoro | 8,10-difluoro | piperazinyl |
| 2. piperazine | 9,10-methylenedioxy | 9,10-methylenedioxy | piperazinyl |
| 3. piperazine | 10-hydroxy | 10-hydroxy | piperazinyl |
| 4. piperazine | 10-methyl | 10-methyl | piperazinyl |
| 5. 4-methylpiperazine | 8,10-difluoro | 8,10-difluoro | 4-methylpiperazinyl |
| 6. 4-methylpiperazine | 9,10-methylenedioxy | 9,10-methylenedioxy | 4-methylpiperazinyl |
| 7. 4-methylpiperazine | 10-hydroxy | 10-hydroxy | 4-methylpiperazinyl |
| 8. 4-methylpiperazine | 10-chloro | 10-chloro | 4-methylpiperazinyl |
| 9. 3-acetamidopyrrolidine | 8,10-difluoro | 8,10-difluoro | 3-aminopyrrolidinyl |
| 10. 3-acetamidopyrrolidine | 9,10-methylenedioxy | 9,10-methylenedioxy | 3-aminopyrrolidinyl |
| 11. 3-acetamidopyrrolidine | 10-hydroxy | 10-hydroxy | 3-aminopyrrolidinyl |
| 12. 3-acetamidopyrrolidine | 9-fluoro | 9-fluoro | 3-aminopyrrolidinyl |
| 13. 3-methylacetamidopyrrolidine | 8,10-difluoro | 8,10-difluoro | 3-methylaminopyrrolidinyl |
| 14. 3-methylacetamidopyrrolidine | 9,10-methylenedioxy | 9,10-methylenedioxy | 3-methylaminopyrrolidinyl |
| 15. 3-methylacetamidopyrrolidine | 10-hydroxy | 10-hydroxy | 3-methylaminopyrrolidinyl |
| 16. 3-methylacetamidopyrrolidine | 10-dimethylamino | 10-dimethylamino | 3-methylaminopyrrolidinyl |
| 17. pyrrolidine | 10-methoxy | 10-methoxy | pyrrolidinyl |
| 18. 3-dimethylaminopyrrolidine | 9,10-methylenedioxy | 9,10-methylenedioxy | 3-dimethylaminopyrrolidinyl |
| 19. 3-dimethylaminopyrrolidine | 8,10-difluoro | 8,10-difluoro | 3-dimethylaminopyrrolidinyl |
| 20. 3-hydroxypyrrolidine | 9-fluoro | 9-fluoro | 3-hydroxypyrrolidinyl |

TABLE II (continued)

| Piperazine replacement | Compound II used ($R_1$ = H, X = F) | Compound I obtained ($R_1$ = H) | |
|---|---|---|---|
| ZH | $R_2$ | $R_2$ | Z |
| 21. piperidine | 10-hydroxy | 10-hydroxy | piperidinyl |
| 22. morpholine | 10-chloro | 10-chloro | morpholinyl |
| 23. thiomorpholine | 10-methyl | 10-methyl | thiomorpholinyl |
| 24. N,N-dimethylhydrazine | 10-methoxy | 10-methoxy | N,N-dimethylhydrazyl |
| 25. dimethylamine | 10-chloro | 10-chloro | dimethylamino |
| 26. homopiperazine | 8,10-difluoro | 8,10-difluoro | homopiperazinyl |
| 27. 2,6-dimethylpiperazine | 9,10-methylenedioxy | 9,10-methylenedioxy | 3,5-dimethylpiperazinyl |

The compound wherein W and Z form a methylenedioxy bridge may be prepared in accordance with the following reaction scheme, in which $R_2$ is as described above and X can be independently identical or non-identical halogen.

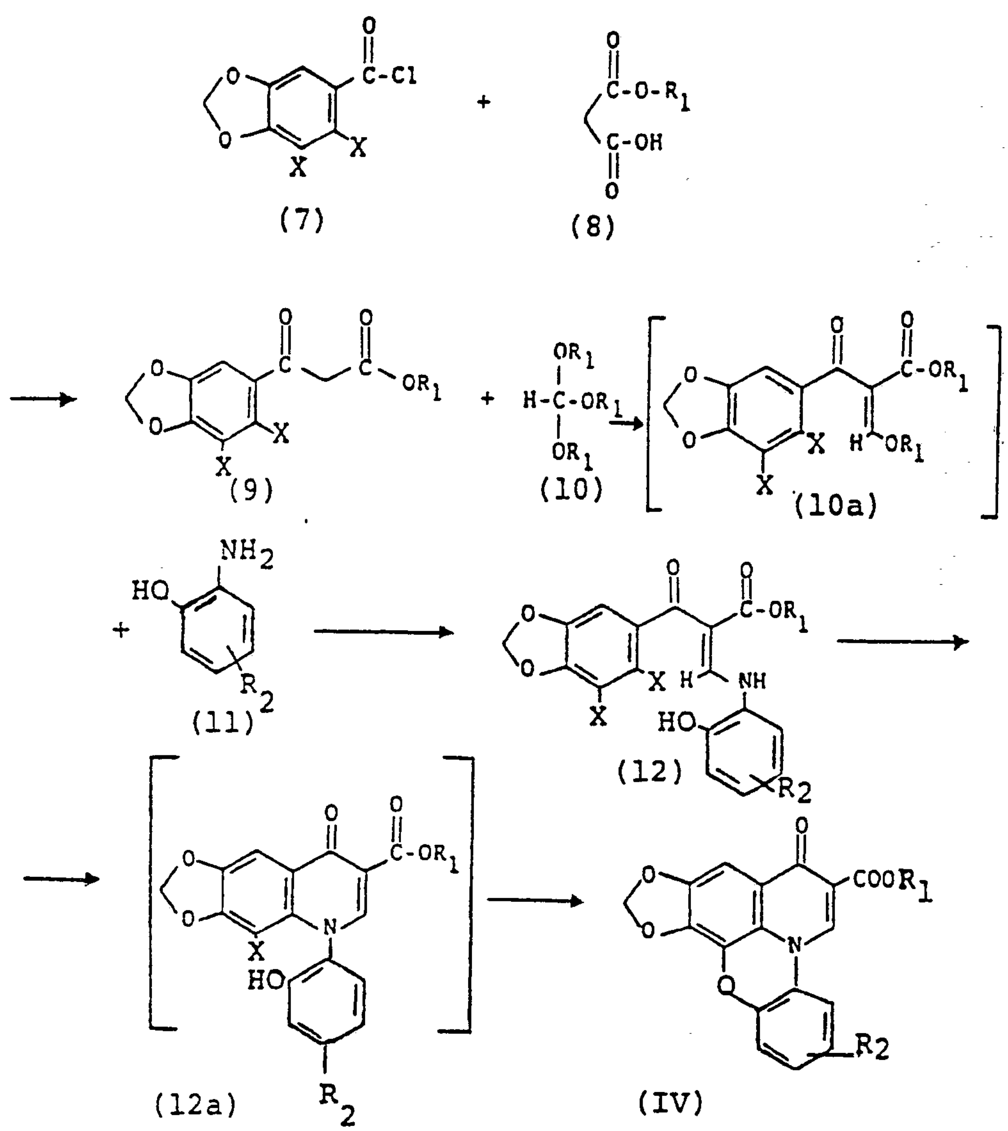

(7) (8) (9) (10) (10a) (11) (12) (12a) (IV)

In accordance with the foregoing reaction scheme, 2,3-dibromo-4,5-methylenedioxybenzoyl chloride is reacted with malonic acid halfester (8) in the presence of N-butyllithium to give the β-ketoester (9). The β-ketoester (9) is then treated with a trialkylorthoformate (10) in the presence of an acid anhydride, followed by reaction with substituted or unsubstituted 2-hydroxyaniline (11) to obtain the enaminoketoester (12). This enaminoketoester (12) is then cyclized by treatment with a strong base such as sodium hydride to obtain the 1,2-methylenedioxy-4-oxo-4H-quino[2,3,4-i,j][1,4]benoxazine-5-carboxylic acid ester (IV) ($R_1$ = alkyl). The ester (IV) is subjected to hydrolysis either with hydrochloric acid or sodium hydroxide to form the free acid (IV) ($R_1$ = H). As used in the following examples, the references to compounds such as (7), (8), (9), etc. and to the substituents, such as R, $R_1$, $R_2$ etc. refer to the corresponding compounds and substituents in the foregoing reaction scheme and in formula IV.

Example 21

1,2-methylenedioxy-4-oxo-4H-quino[2,3,4-i,j][1,4]benoxazine-5-carboxylic acid

(a) To a dry ice cooled solution of 0.85 g malonic acid monoethyl ester in 25 ml of tetrahydrofuran (THF) is slowly added 9.2 ml of 1.4 M n-butyl lithium in THF and the flask is warmed to −5°C. After 5 minutes, the solution is cooled back to −70°C. After 1.3 g of the acid chloride (7) (X = Br) is added, the cooling bath is removed and warmed up to room temperature over an hour. The solution is then partitioned between 1 N HCl and ether. The ether portion is washed with $NaHCO_3$ and is dried over $MgSO_4$, then evaporated to obtain a pale yellow oil which is then purified over a silica gel column to yield β-ketoester (9) ($R_1$ = $C_2H_5$, X = Br).

(b) To a solution of 1.25 g of β-ketoester (9) ($R_1 = C_2H_5$, X = Br) in 0.8 ml of triethylorthoformate and 5 ml of acetic anhydride is heated at 135°C for 1½ hours with the removal of the ethyl acetate formed during the reaction. The solution is evaporated under reduced pressure to a mobile oil. The oil is then dissolved in 5 ml of methylene chloride and 0.41 g of 2-hydroxy-aniline is added into the solution. After 1 hour, the solution is evaporated to dryness and crystallized from ethylacetate yielding (12), wherein $R_1 = C_2H_5$, X = Br and $R_2$ = H.

(c) To a solution of 3.6 g of the preceding product (12), ($R_1 = C_2H_5$, $R_2$ = H, X = Br) in 30 ml dimethoxyethane is slowly added 0.56 g of a 60% sodium hydride-in-oil suspension. The mixture is refluxed for 20 hours and is cooled and diluted with water to a volume of 100 ml. The mixture is then filtered and the solid is washed with a 1:1 hexane/ether solution to obtain (IV) ($R_1 = C_2H_5$, $R_2$ = H).

(d) To a suspension of 1.6 g of (14) ($R_1 = C_2H_5$, $R_2$ = H) in 20 ml THF is added a sodium hydroxide solution (0.2 g in 20 ml water). The mixture is heated at 80°C for 2 hours resulting in a clear solution which is evaporated under reduced pressure to dryness. The solid is dissolved in 200 ml water and 1 ml acetic acid is added. The resulting precipitate is filtered and washed with cold water to produce 1,2-methylenedioxy-4-oxo-4H-quino[2,3,4-i,j][1,4]benoxazine-5-carboxylic acid (IV) ($R_1$ = H, $R_2$ = H).

Example 22

1,2-methylenedioxy-10-fluoro-4-oxo-4H-quino[2,3,4-i,j][1,4]benoxazine-5-carboxylic acid

The procedure of Example 21 can be repeated replacing 2-hydroxyaniline in 21(b) with 2-hydroxy-4-fluoroaniline to obtain 1,2-methylenedioxy-10-fluoro-4-oxo-4H-quino[2,3,4-i,j][1,4]benoxazine-5-carboxylic acid (IV), $R_1$ = H, $R_2$ = 10-fluoro.

Example 23

In the described fashion as Example 21, replacing 2-hydroxyaniline in Example 21(b) with appropriate substituted 2-hydroxyaniline, one can obtain the additional substituted 1,2-methylenedioxy-4-oxo-4H-quino[2,3,4-i,j][1,4]benoxazine-5-carboxylic acid (IV), ($R_1$ = H) as listed in Table III.

TABLE III

| Substituted 2-hydroxyaniline | Compound IV obtained, ($R_1$ = H) |
|---|---|
| $R_2$ = 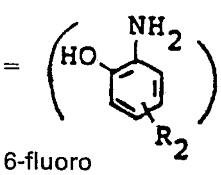 | $R_2$ = |
| (a) 6-fluoro | 8-fluoro |
| (b) 5-fluoro | 9-fluoro |
| (c) 3-fluoro | 11-fluoro |
| (d) 4,6-difluoro | 8,10-difluoro |
| (e) 4-chloro | 10-chloro |
| (f) 4-methyl | 10-methyl |
| (g) 4,5-methylenedioxy | 9,10-methylenedioxy |
| (h) 4-hydroxy | 10-hydroxy |
| (i) 4-methoxy | 10-methoxy |

The compounds wherein X is a pyridine ring may be prepared in accordance with the following reaction scheme:

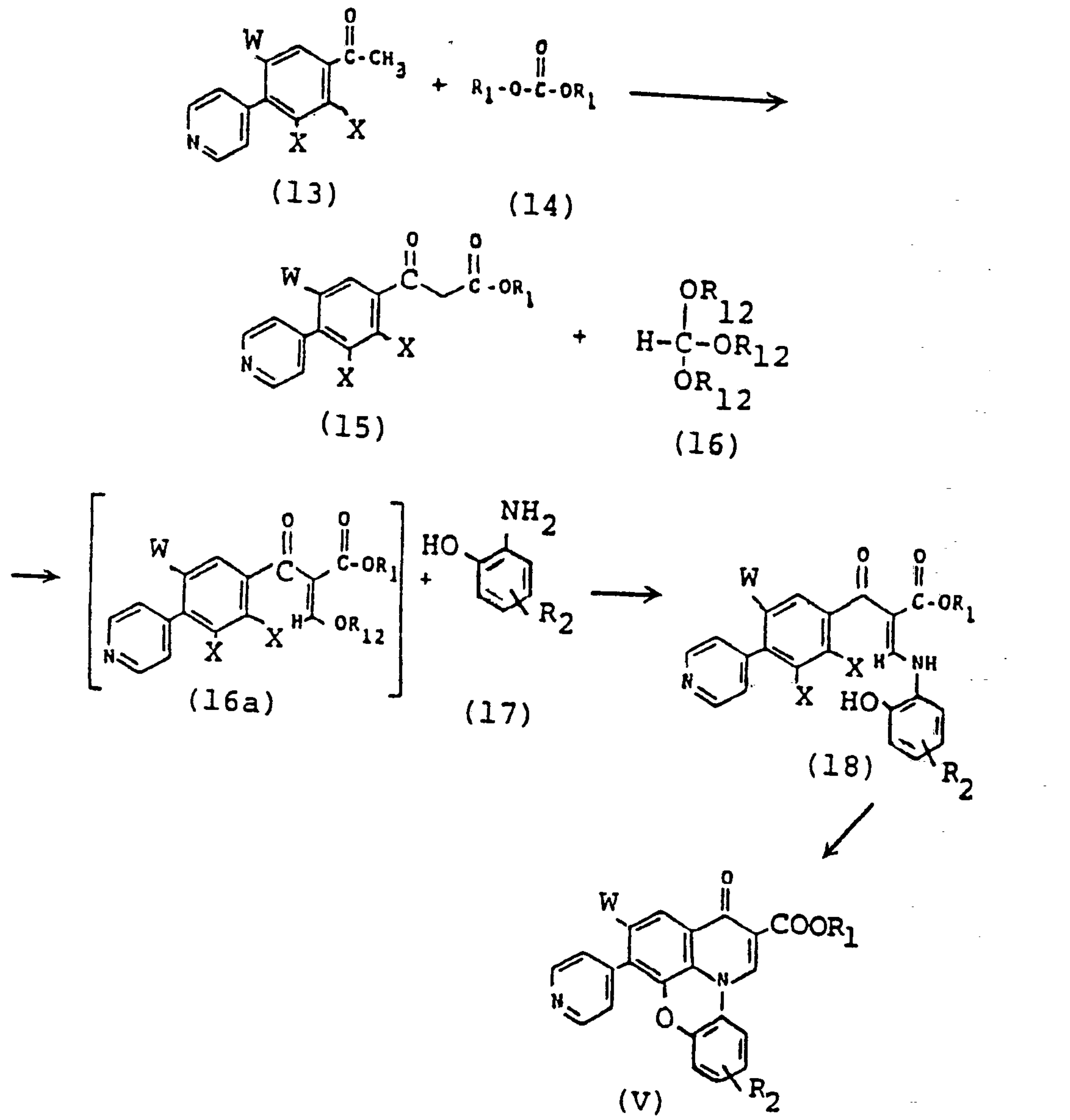

(13) (14) (15) (16) (16a) (17) (18) (V)

In accordance with the foregoing reaction scheme wherein X is a halogen or a leaving group, the acetophenone (13) is reacted with a dialkoxycarbonate (14) in the presence of a strong base to obtain the corresponding β-ketoester (15). In the dialkoxycarbonate (14), $R_1$ may be an alkyl group of, for example, 1 to 10 carbon atoms, but is preferably loweralkyl, such as ethyl. Suitable bases include metal hydrides, such as sodium hydride, potassium hydride and the like, as well as metal alkoxides in alcohol, such as sodium ethoxide in ethanol. The preferred base is sodium hydride. Formation of the β-ketoester (15) is facilitated by reacting the acetophenone (13) with the dialkoxycarbonate (14) at elevated temperatures, such as from about 20°C to about 120°C, and preferably from about 30°C to about 90°C until completion of the reaction. The β-ketoester may then be separated from the reaction mixture in a conventional manner.

The β-ketoester (15) is then treated with a trialkylorthoformate (16) in the presence of an acid anhydride, preferably acetic anhydride, followed by reaction with substituted or unsubstituted 2 hydroxy-aniline (17) to obtain the enamino-ketoester (18). In the trialkylorthoformate (16), $R_{12}$ may be an alkyl group of, for example, from 1 to 10 carbon atoms, but is preferably loweralkyl, such as ethyl. Reaction with the trialkylorthoformate is preferably conducted at elevated temperatures, such as from about 50°C to about 150°C, and preferably from about 100°C to about 140°C, to obtain an oily liquid, which may be isolated or unisolated, as desired (shown in brackets in the reaction scheme). Reaction of the latter with the substituted or unsubstituted 2-hydroxyaniline (17) is preferably conducted in an appropriate aprotic or non-aprotic solvent, preferably methylene chloride or tetrahydrfuran, and may be conducted at room or suitable elevated temperatures, as desired.

The enamino-ketoester (18) is then cyclized, such as by treatment with a strong base as defined above, preferably sodium hydride, to obtain the 1-(4-pyridyl)-4-oxo-4H-quino[2,3,4-i,j][1,4]benoxazine-5-carboxylic acid ester (V) ($R_1$ = alkyl). Cyclization is conducted in the presence of an aprotic solvent, such as dimethoxyethane, dimethylformamide, tetrahydrofuran or chlorobenzene, and is preferably conducted at temperatures of about 20°C, to about 145°C, and more preferably at the reflux temperature of the solvent employed.

The ester (V) ($R_1$ = alkyl) is subjected to hydrolysis such as by treatment with sodium hydroxide, or mineral acid, to form the free acid (V).

The 1-(4-pyridyl)-4-oxo-4H-quino[2,3,4-i,j][1,4]benoxazine-5-carboxylic acid (V) ($R_1$ = H) can be converted into the corresponding ester, if desired, by conventional esterification procedures, such as by treating the free acid (V) with the appropriate alcohol in the presence of an acid catalyst, by converting the free acid (V) into the corresponding acid chloride followed by displacement of the chloro radical with the appropriate alcohol, or by treating the sodium salt of the acid (V) with a suitable reactive halide, such as chloromethylpivalate in dimethoxyethane to obtain, for example, the pivaloyloxymethyl ester.

The foregoing may be better understood from the following examples, which are presented for purposes of illustration and are not intended to limit the scope of the inventive concepts. As used in the following examples, the references to compounds, such as (13), (14), (15), etc., and to substituents, such as $R_1$, $R_2$, etc., refer to the corresponding compounds and substituents in the foregoing reaction scheme and in formula V.

Example 24

1-(4-pyridyl)-2-fluoro-4-oxo-4H-quino[2,3,4-i,j][1,4]benzoxazine-5-carboxylic acid

(a) A cold solution of 28.4 g 2,3-dichloro-4-(4-pyridyl)-5-fluoroacetophenone in 350 ml diethylcarbonate is slowly added to 8.0 g 60% sodium hydride-in-oil suspension. The mixture is heated at 80°C for 3 hours, then poured into 700 ml ice cold water solution containing 25 ml acetic acid. The mixture is extracted with three 400 ml portions of ether. The organic phase is dried over $MgSO_4$, evaporated and the obtained oil is purified through silica gel column to give pure (15). (X = Cl, $R_1$ = $C_2H_5$, W = F).

(b) A solution of 16.6 g of β-ketoester (15) ($R_1$ = $C_2H_5$, X = Cl, W = F) in 14 ml of triethylorthoformate and 35 ml of acetic anhydride is heated at 135°C for 1½ hours with the removal of the ethyl acetate formed during the reaction. The solution is evaporated under reduced pressure to a mobile oil. The oil is then dissolved in 150 ml of methylene chloride and 7.5 g of 2-hydroxyaniline is added into the solution. After 1 hour, the solution is evaporated to dryness yielding (18), wherein $R_1$ = $C_2H_5$, X = Cl, $R_2$ = H, W = F.

(c) To a cold solution of 14 g of the preceding product (18) ($R_1$ = $C_2H_5$, $R_2$ = H, X = Cl, W = F), in 140 ml. tetrahydrofuran (THF) is slowly added 2.5 g of a 60% sodium hydride-in-oil suspension. The mixture is refluxed for 6 hours and is cooled and diluted with water to a volume of 1.5 liters. The mixture is then filtered and the solid is washed with 1:1 hexane/ether solution to obtain (V) wherein $R_1$ = $C_2H_5$, W = F, $R_2$ = H).

(d) To a suspension of 5 g of (V) ($R_1$ = $C_2H_5$, W = F, $R_2$ = H) in 30 ml of THF is added a sodium hydroxide solution (0.63 g in 10 ml water). The mixture is heated at 80°C for 2 hours resulting in a clear solution which is evaporated under reduced pressure to dryness. The solid is dissolved in 200 ml water, and 2 ml acetic acid is added. The resulting precipitate is filtered and washed with cold water, to produce 1-(4-pyridyl)-2-fluoro-4-oxo-4H-quino[2,3,4-i,j][1,4]benoxazine-5-carboxylic acid (V) ($R_1$ = H, $R_2$ = H, W = F).

Example 25

1-(4-pyridyl)-2,10-difluoro-4-oxo-4H-quino[2,3,4-i,j][1,4]benoxazine-5-carboxylic acid

The procedure of Example 24 can be repeated replacing 2-hydroxyaniline with 2-hydroxy-4-fluoroaniline in Example 24(b) to obtain 1-(4-pyridyl)-2,10-difluoro-4-oxo-4H-quino[2,3,4-i,j][1,4]benoxazine-5-carboxylic acid (I), ($R_1$ = H, $R_2$ = 10-fluoro, W = F).

Example 26

In the described fashion as Example 24 replacing 2-hydroxyaniline in Example 24(b) with 2,4-dihydroxy-aniline, one can obtain 1-(4-pyridyl)-2-fluoro-10-hydroxy-4-oxo-4H-quino[2,3,4-i,j][1,4]-benoxazine-5-carboxylic acid (V) ($R_1$ = H, $R_2$ = 10-hydroxy, W = F).

Example 27

The procedure of Example 24 can be repeated replacing 2-hydroxyaniline in Example 24(b) with 2-hydroxy-4-methoxyaniline to obtain 1-(4-pyridyl)-2-fluoro-10-methoxy-4-oxo-4H-quino[2,3,4-i,j][1,4]-benoxazine-5-carboxylic acid (V), ($R_1$ = H, $R_2$ = 10-methoxy, W = F).

Example 28

In the described fashion as Example 24 replacing 2-hydroxyaniline in Example 24(b) with various substituted 2-hydroxyaniline, one can obtain additional substituted 1-(4-pyridyl)-2-fluoro-4-oxo-4H-quino-[2,3,4-i,j][1,4]benoxazine-5-carboxylic acid (V) as summarized in Table IV.

TABLE IV

| 2-Hydroxyaniline Replacement | Compound V obtained ($W = F$, $R_1 = H$) |
|---|---|
| $R_2$ = 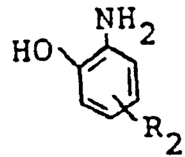 | $R_2$ = |
| (a) 6-fluoro | 8-fluoro |
| (b) 5-fluoro | 9-fluoro |
| (c) 3-fluoro | 11-fluoro |
| (d) 4,6-difluoro | 8,10-difluoro |
| (e) 4-chloro | 10-fluoro |
| (f) 4-methyl | 10-methyl |
| (g) 4,5-methylenedioxy | 9,10-methylenedioxy |

Example 29

In the described fashion as Example 24 replacing 2,3-dichloro-4-(4-pyridyl)-5-fluoroacetophenone in Example 24(a) with 2,3-dichloro-4-(4-pyridyl)aceto- phenone and optionally 2-hydroxyaniline in Example 24(b) with 2-hydroxy-4-fluoroaniline or 2-hydroxy-4,5-methylenedioxyaniline, one can obtain the following three compounds:

(a) 1-(4-pyridyl)-4-oxo-4H-quino[2,3,4-i,j][1,4]benoxazine-5-carboxylic acid (V) ($R_1 = H$, $W = H$, $R_2 = H$).

(b) 1-(4-pyridyl)-10-fluoro-4-oxo-4H-quino[2,3,4-i,j][1,4]benoxazine-5-carboxylic acid (V) ($R_1 = H$, $W = H$, $R_2$ = 10-fluoro).

(c) 1-(4-pyridyl)-9,10-methylenedioxy-4-oxo-4H-quino[2,3,4-i,j][1,4]benoxazine-5-carboxylic acid (V) ($R_1 = H$, $W = H$, $R_2$ = 9,10-methylenedioxy).

It will be understood that various changes and modifications can be made in the details of formulation, procedure and use without departing from the spirit of the invention, especially as defined in the following claims.

**Claims**

1. A compound having the formula

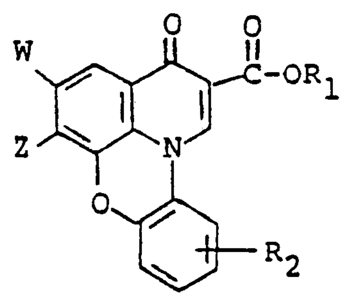

(IA)

wherein $R_1$ is hydrogen or a carboxy protecting group; $R_2$ is one or more groups selected from the group consisting of hydrogen, halogen, nitro, carboxyl, methylenedioxy, cyano, $C_1$ to $C_6$ alkyl and substituted derivatives thereof, wherein the substituents are selected from hydroxy and halo; a group having the formula:

$$-Y-R_3$$

wherein $-Y-$ is $-O-$ or $-S-$ and $R_3$ is hydrogen or $C_1$ to $C_6$ alkyl, and an amine group having the formula:

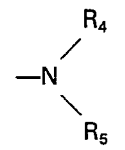

wherein $R_4$ and $R_5$ are independently hydrogen or $C_1$ to $C_6$ alkyl; W is halogen or hydrogen; and Z is selected from the group consisting of an aliphatic heterocyclic ring having the formula

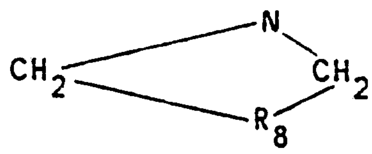

wherein $R_8$ is dimethylene or a group of the formula $—CH_2—R_9—CH_2—$ wherein $R_9$ is selected from the group consisting of —S—, —O—, —NH—, $—CH_2—$ and $—CH_2—NH—$; substituted derivates thereof wherein one or more substituents are selected from the group consisting of $C_1$ to $C_6$ alkyl, hydroxy, alkanoyl containing 1 to 6 carbon atoms, alkanoylamido containing 1 to 6 carbon atoms and an amine of the formula

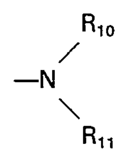

wherein $R_{10}$ and $R_{11}$ are each independently selected from the group consisting of hydrogen, $C_1$ to $C_6$ alkyl and substituted derivatives thereof, wherein the substituents are selected from hydroxy and halo; an amino group of the formula

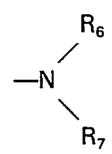

wherein $R_6$ is hydrogen or $C_1$ to $C_{10}$ alkyl or substituted derivatives thereof wherein the substituents are selected from hydroxy and halo; and $R_7$ is selected from the groups consisting of $C_1$ to $C_{10}$ alkyl and substituted derivatives thereof wherein the substituents are selected from hydroxy and halo, and furthermoire consisting of an amine group, a mono-($C_1$ to $C_6$) alkylamino group and a di-($C_1$ to $C_6$) alkylamino group; a pyridine ring of the formula

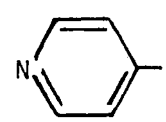

or substituted derivatives of the pyridine ring wherein the substituents are selected from the group consisting of $C_1$ to $C_6$ alkyl, halogen, a group of the formula $—Y—R_{12}$ wherein Y is —O— or —S— and $R_{12}$ is $C_1$ to $C_6$ alkyl, hydroxy, alkanoyl containing 1 to 6 carbon atoms, alkanoylamido containing 1 to 6 carbon atoms and an amine of the formula:

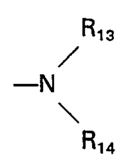

wherein $R_{13}$ and $R_{14}$ are each independently selected from the group consisting of hydrogen, $C_1$ to $C_6$ alkyl and substituted derivatives thereof wherein the substituents are selected from hydroxy and halo; or, alternatively, W and Z together may form a methylenedioxy bridge, and pharmaceutically acceptable salts thereof.

2. A compound as defined in Claim 1 wherein W is F; Z is piperazinyl, (4-methyl)-piperazinyl, (3,5-dimethyl)-piperazinyl, (3-amino)-pyrrolidinyl, (3-methylamino)-pyrrolidinyl, (3-dimethylamino)-pyrrolidinyl, (3-acetamido)-pyrrolidinyl, (3-methylacetamido)-pyrrolidinyl or (3-hydroxy)-pyrrolidinyl, and $R_2$ is one or more of a $C_1$ to $C_6$ alkyl group, a hydrogen group, a halogen group, a carboxy group and a methylenedioxy group, and pharmaceutically acceptable salts thereof.

3. A compound as defined in Claim 2 wherein $R_2$ is hydrogen; Z is piperazinyl or 4-methylpiperazinyl; and $R_1$ is hydrogen.

4. A compound as defined in Claim 2 wherein $R_2$ is 10-fluoro or 8,10-difluoro; Z is piperazinyl, 4-methylpiperazinyl, 3-aminopyrrolidinyl, 3-methylaminopyrrolidinyl or dimethylaminopyrrolidinyl; and $R_1$ is hydrogen.

5. A compound as defined in Claim 1 wherein W and Z together form a methylenedioxy bridge; $R_1$ is hydrogen or a carboxy protecting group; $R_2$ is one or more groups selected from the group consisting of hydrogen, halogen, nitro, methylenedioxy, carboxyl, cyano, $C_1$ to $C_6$ alkyl and substituted derivatives thereof, wherein the substituents are selected from hydroxy and halo; a group having the formula:

$$—Y—R_3$$

wherein —Y— is —O— or —S— and $R_3$ is hydrogen or $C_1$ to $C_6$ alkyl, and an amine group having the formula:

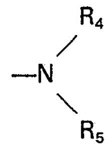

wherein $R_4$ and $R_5$ are independently hydrogen or $C_1$ to $C_6$ alkyl; and, pharmaceutically acceptable salts thereof.

6. A compound as defined in Claim 5 wherein $R_2$ is hydrogen, $C_1$ to $C_6$ alkyl, 10-fluoro, methylenedioxy, or 8,10-difluoro group; and $R_1$ is hydrogen.

7. A compound as defined in Claim 1 wherein $R_1$ is hydrogen or a carboxy protecting group; W is halogen or hydrogen; $R_2$ is one or more groups selected from the group consisting of hydrogen, halogen, nitro, carboxyl, methylenedioxy, cyano, $C_1$ to $C_6$ alkyl and substituted derivatives thereof, wherein the substituents are selected from hydroxy and halo, a group having the formula:

$$—Y—R_3$$

wherein —Y— is —O— or —S— and $R_3$ is hydrogen or $C_1$ to $C_6$ alkyl, and an amine group having the formula:

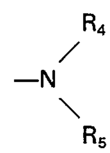

wherein $R_4$ and $R_5$ are independently hydrogen or $C_1$ to $C_6$ alkyl; and Z is a 4-pyridyl group pharmaceutically acceptable salts thereof.

8. A compound as defined in Claim 7 wherein $R_2$ is hydrogen, fluoro, difluoro or methylenedioxy; $R_1$ is hydrogen, and W is fluoro or hydrogen.

**Patentansprüche**

1. Verbindung der Formel

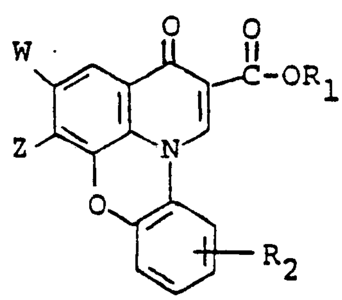

(IA)

worin $R_1$ Wasserstoff oder eine Carboxyschutzgruppe ist; $R_2$ ist eine oder mehrere Gruppen, ausgewählt aus der Gruppe, bestehend aus Wasserstoff, Halogen, Nitro, Carboxyl, Methylendioxy, Cyano, $C_1$—$C_6$-Alkyl und substituierten Derivaten derselben, worin die Substituenten ausgewählt sind aus Hydroxy und Halogen; eine Gruppe von der Formel —Y—$R_3$, worin —Y— —O— oder —S— und $R_3$ Wasserstoff oder $C_1$—$C_6$-Alkyl ist, und eine Amingruppe der Formel

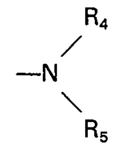

ist, worin $R_4$ und $R_5$ unabhängig Wasserstoff oder $C_1$—$C_6$-Alkyl sind; W ist Halogen oder Wasserstoff; und Z ist ausgewählt aus der Gruppe, bestehend aus einem aliphatischen heterocyclischen Ring der Formel

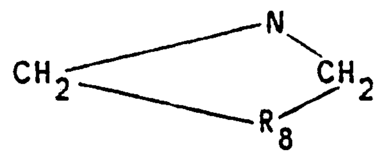

worin $R_8$ Dimethylen oder eine Gruppe der Formel —$CH_2$—$R_9$—$CH_2$— ist, worin $R_9$ ausgewählt wird aus der Gruppe, bestehend aus —S—, —O—, —NH—, —$C_2$— und —$CH_2$—NH—; substituierten Derivaten derselben, worin einer oder mehrere Substituenten ausgewählt sind aus der Gruppe, bestehend aus $C_1$—$C_6$-Alkyl, Hydroxy, Alkanoyl, enthaltend 1 bis 6 C-Atome, Alkanoylamido, enthaltend 1 bis 6 C-Atome und einem Amin der Formel

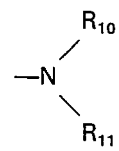 ,

worin $R_{10}$ und $R_{11}$ jeweils unabhängig ausgewählt sind aus der Gruppe, bestehend aus Wasserstoff, $C_1$—$C_6$-Alkyl und substituierten Derivaten derselben, wobei die Substituenten ausgewählt sind aus Hydroxy und Halogen; einer Aminogruppe der Formel

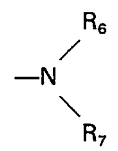 ,

worin $R_6$ Wasserstoff oder $C_1$—$C_{10}$-Alkyl oder substituierten Derivaten derselben ist, worin die Substituenten ausgewählt sind aus Hydroxy und Halogen; und $R_7$ ausgewählt wird aus den Gruppen, bestehend aus $C_1$—$C_{10}$-Alkyl und substituierten Derivaten derselben, wobei die Substituenten ausgewählt sind aus Hydroxy und Halogen, und ferner bestehend aus einer Amingruppe, einer Mono-($C_1$—$C_6$)-Alkylaminogruppe und einer Di-($C_1$—$C_6$)-Alkylaminogruppe; einem Pyridinring der Formel

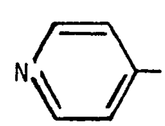

oder substituierten Derivaten des Pyridinringes, worin die Substituenten ausgewählt sind aus der Gruppe, bestehend aus $C_1$—$C_6$-Alkyl, Halogen, einer Gruppe der Formel —Y—$R_{12}$, worin Y —O— oder —S— und $R_{12}$ $C_1$—$C_6$-Alkyl, Hydroxy, Alkanoyl, enthaltend 1 bis 6 C-Atome, Alkanoylamido, enthaltend 1 bis 6 C-Atome, und ein Amin der Formel

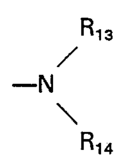

ist, worin $R_{13}$ und $R_{14}$ jeweils unabhängig ausgewählt werden aus der Gruppe, bestehend aus Wasserstoff, $C_1$—$C_6$-Alkyl und substituierten Derivaten derselben, wobei die Substituenten ausgewählt sind aus Hydroxy und Halogen; oder, alternativ, W und Z zusammen eine Methylendioxybrücke bilden können und pharmazeutisch akzeptable Salze derselben.

2. Verbindung wie in Anspruch 1 definiert, worin W F ist; Z Piperazinyl, (4-Methyl)-piperazinyl, (3,5-Dimethyl)-piperazinyl, (3-Amino)-pyrrolidinyl, (3-Methylamino)-pyrrolidinyl, (3-Dimethylamino)-pyrrolidinyl, (3-Acetamido)-pyrrolidinyl, (3-Methylacetamido)-pyrrolidinyl oder (3-Hydroxy)-pyrrolidinyl ist, und $R_2$ eine oder mehrere einer $C_1$—$C_6$-Alkylgruppe, einer Wasserstoffgruppe, einer Halogengruppe, einer Carboxygruppe und einer Methylendioxygruppe und pharmazeutisch akzeptablen Salze derselben ist.

3. Verbindung wie in Anspruch 2 definiert, worin $R_2$ Wasserstoff, Z Piperazinyl oder 4-Methylpiperazinyl und $R_1$ Wasserstoff ist.

4. Verbindung wie in Anspruch 2 definiert, in welcher $R_2$ 10-Fluor oder 8,10-Difluor, Z Piperazinyl, 4-Methylpiperazinyl, 3-Aminopyrrolidinyl, 3-Methylaminopyrrolidinyl oder Dimethylaminopyrrolidinyl und $R_1$ Wasserstoff ist.

5. Verbindung wie in Anspruch 1 definiert, worin W und Z zusammen eine Methylendioxybrücke bilden; $R_1$ Wasserstoff oder eine Carboxyschutzgruppe ist und $R_2$ eine oder mehrere Gruppen, ausgewählt aus der Gruppe, bestehend aus Wasserstoff, Halogen, Nitro, Methylendioxy, Carboxyl, Cyano, $C_1$—$C_6$-Alkyl und substituierten Derivaten derselben, worin die Substituenten ausgewählt sind aus Hydroxy und Halogen; einer Gruppe der Formel —Y—$R_3$, worin —Y —O— oder —S— und $R_3$ Wasserstoff oder $C_1$—$C_6$-Alkyl ist, und eine Amingruppe der Formel

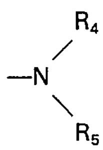 ,

worin $R_4$ und $R_5$ unabhängig Wasserstoff oder $C_1$—$C_6$-Alkyl und pharmazeutisch akzeptable Salze derselben ist.

6. Verbindung wie in Anspruch 5 definiert, worin $R_2$ Wasserstoff, $C_1$—$C_6$-Alkyl, 10-Fluor, Methylendioxy oder 8,10-Difluor und $R_1$ Wasserstoff ist.

7. Verbindung wie in Anspruch 1 definiert, worin $R_1$ Wasserstoff oder eine Carboxyschutzgruppe, W Halogen oder Wasserstoff, $R_2$ eine oder mehrere Gruppen ist, ausgewählt aus der Gruppe, bestehend aus Wasserstoff, Halogen, Nitro, Carboxyl, Methylendioxy, Cyano, $C_1$—$C_6$-Alkyl und substituierten Derivaten derselben, worin die Substituenten ausgewählt sind aus Hydroxy und Halogen und einer Gruppe der Formel —Y—$R_3$, worin —Y— —O— oder —S— und $R_3$ Wasserstoff oder $C_1$—$C_6$-Alkyl ist, und eine Amingruppe der Formel

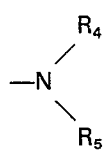 ,

worin $R_4$ und $R_5$ unabhängig Wasserstoff oder $C_1$—$C_6$-Alkyl und Z eine 4-Pyridylgruppe sind und pharmazeutisch akzeptable Salze.

8. Verbindung wie in Anspruch 7 definiert, worin $R_2$ Wassertoff, Fluor, Difluor oder Methylendioxy bedeutet und $R_1$ Wasserstoff und W Fluor oder Wasserstoff ist.

**Revendications**

1. Un composé ayant la formule suivante:

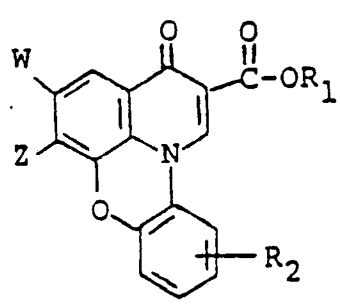

(IA)

dans laquelle $R_1$ est un atome d'hydrogène ou un groupe protecteur du groupe carboxy; $R_2$ est un ou

plusieurs groupes choisis dans le groupe comprenant un atome d'hydrogène, d'halogène, un groupe nitro, carboxyle, méthylènedioxy, cyano, $C_1$—$C_6$ alkyle et les dérivés substitués de ce dernier, dans lesquels les substituants sont choisis parmi les groupes hydroxy et halo; un groupe ayant la formule:

$$—Y—R_3$$

dans laquelle —Y— est —O— ou —S— et $R_3$ est un atome d'hydrogène ou un groupe $C_1$—$C_6$ alkyle, et un groupe amino ayant la formule:

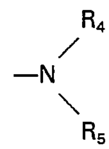

dans laquelle $R_4$ et $R_5$ sont indépendamment un atome d'hydrogène ou un groupe $C_1$—$C_6$ alkyle; W est un atome d'halogène ou d'hydrogène; et Z est choisi dans le groupe comprenant un noyau hétérocyclique aliphatique ayant la formule

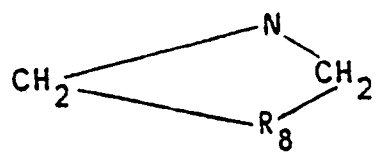

dans laquelle $R_8$ est un groupe diméthylène ou un groupe de formule $—CH_2—R_9—CH_2—$ dans laquelle $R_9$ est choisi dans le groupe comprenant —S—, —O—, —NH—, $—CH_2—$ et $—CH_2—NH—$; les dérivés substitués de ce dernier dans lesquels un ou plusieurs substituants sont choisis dans le groupe comprenant un groupe $C_1$—$C_6$ alkyle, hydroxy, alcanoyle contenant 1 à 6 atomes de carbone, alcanoylamido contenant 1 à 6 atomes de carbone et un groupe amino de formule:

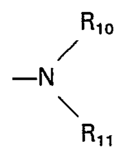

dans laquelle $R_{10}$ et $R_{11}$ sont chacun indépendamment choisis dans le groupe comprenant un atome d'hydrogène, un groupe $C_1$—$C_6$ alkyle et les dérivés substitués de ce dernier, dans lesquels les substituants sont choisis parmi les groupes hydroxy et halo; un groupe amino de formule:

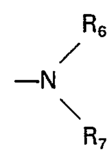

dans laquelle $R_6$ est un atome d'hydrogène ou un groupe $C_1$—$C_{10}$ alkyle ou les dérivés substitués de ce dernier dans lesquels les substituants sont choisis parmi les groupes hydroxy et halo; et $R_7$ est choisi parmi les groupes constitués de $C_1$—$C_{10}$ alkyle et les dérivés substitués de ces derniers dans lesquels les substituants sont choisis parmi les groupes hydroxy et halo, et constitué en outre par un groupe amine, un groupe mono-($C_1$—$C_6$) alkylamino et un groupe di-($C_1$—$C_6$) alkylamino; un noyau pyridine de formule

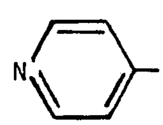

ou les dérivés substitués du noyau pyridine dans lesquels les substituants sont choisis dans le groupe comprenant un groupe $C_1$—$C_6$ alkyle, un atome d'halogène, un groupe de formule $—Y—R_{12}$ dans laquelle Y est —O— ou —S— et $R_{12}$ est un groupe $C_1$—$C_6$ alkyle, hydroxy, alcanoyle contenant de 1 à 6 atomes de carbone, alcanoylamido contenant 1 à 6 atomes de carbone et une amine de formule:

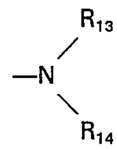

dans laquelle $R_{13}$ et $R_{14}$ sont chacun choisis indépendamment dans le groupe comprenant un atome d'hydrogène, un groupe $C_1$—$C_6$ alkyle et les dérivés substitués de ce dernier dans lesquels les substituants sont choisis parmi les groupes hydroxy et halo; ou alternativement, W et Z ensemble peuvent former un pont méthylènedioxy, et les sels pharmaceutiquement acceptables dudit composé.

2. Un composé tel que défini dans la revendication 1, selon lequel W est F; Z est un groupe pipérazinyle, (4-méthyl)-pipérazinyle, (3,5-diméthyl)-pipérazinyle, (3-amino)-pyrrolidinyle, (3-méthylamino)-pyrrolidinyle, (3-diméthylamino)-pyrrolidinyle, (3-acétamido)-pyrrolidinyle, (3-méthyl-acétamido)-pyrrolidinyle ou (3-hydroxy)-pyrrolidinyle, et $R_2$ est un ou plusieurs groupes $C_1$—$C_6$ alkyle, un atome d'hydrogène, d'halogène, un groupe carboxy et un groupe méthylènedioxy, et les sels pharmaceutiquement acceptables dudit composé.

3. Un composé tel que défini dans la revendication 2, selon lequel $R_2$ est un atome d'hydrogène; Z est un groupe pipérazinyle ou 4-méthylpipérazinyle; et $R_1$ est un atome d'hydrogène.

4. Un composé tel que défini dans la revendication 2, selon lequel $R_2$ est un groupe 10-fluoro ou 8,10-difluoro; Z est un groupe pipérazinyle, 4-méthylpipérazinyle, 3-aminopyrrolidinyle, 3-méthylamino-pyrrolidinyle ou diméthylaminopyrrolidinyle; et $R_1$ est un atome d'hydrogène.

5. Un composé tel que défini dans la revendication 1, selon lequel W et Z forment ensemble un pont méthylènedioxy; $R_1$ est un atome d'hydrogène ou un groupe protecteur du groupe carboxy; $R_2$ est un ou plusieurs groupes choisis dans le groupe comprenant un atome d'hydrogène, d'halogene, un groupe nitro, méthylènedioxy, carboxyle, cyano, $C_1$—$C_6$ alkyle et les dérivés substitués de ce dernier, dans lesquels les substituants sont choisis parmi les groupes hydroxy et halo; un groupe ayant la formule:

$$—Y—R_3$$

dans laquelle —Y— est —O— ou —S— et $R_3$ est un atome d'hydrogène ou un groupe $C_1$—$C_6$ alkyle, et un groupe amine ayant la formule:

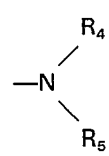

dans laquelle $R_4$ et $R_5$ sont indépendamment un atome d'hydrogène ou un groupe $C_1$—$C_6$ alkyle; et les sels pharmaceutiquement acceptables dudit composé.

6. Un composé tel que défini dans la revendication 5, selon lequel $R_2$ est un atome d'hydrogène, un groupe $C_1$—$C_6$ alkyle, 10-fluoro, méthylènedioxy ou 8,10-difluoro; et $R_1$ est un atome d'hydrogène.

7. Un composé tel que défini dans la revendication 1, selon lequel $R_1$ est un atome d'hydrogène ou un groupe protecteur du groupe carboxy; W est un atome d'halogène ou d'hydrogène; $R_2$ est un ou plusieurs groupes choisis dans le groupe comprenant un atome d'hydrogène, d'halogène, un groupe nitro, carboxyle, méthylènedioxy, cyano, $C_1$—$C_6$ alkyle et les dérivés substitués de ce dernier, dans lesquels les substituants sont choisis parmi les groupes hydroxy et halo; un groupe ayant la formule:

$$—Y—R_3$$

dans laquelle —Y— est —O— ou —S— et $R_3$ est un atome d'hydrogène ou un groupe $C_1$—$C_6$ alkyle, et un groupe amine ayant la formule:

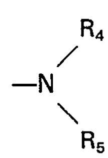

dans laquelle $R_4$ et $R_5$ sont indépendamment un atome d'hydrogène ou un groupe $C_1$—$C_6$ alkyle; et Z est un groupe 4-pyridyle et les sels pharmaceutiquement acceptables dudit composé.

8. Un composé tel que défini dans la revendication 7, selon lequel $R_2$ est un atome d'hydrogène, un groupe fluoro, difluoro ou méthylènedioxy; $R_1$ est un atome d'hydrogène, et W est un groupe fluoro ou un atome d'hydrogène.